# EUROPÄISCHE PATENTSCHRIFT

(11) **EP 2 205 205 B1**
(45) Veröffentlichungstag und Bekanntmachung des Hinweises auf die Patenterteilung: **27.08.2014**
(21) Anmeldenummer: 08847986.0
(22) Anmeldetag: 06.11.2008
(51) Int. Cl.: A61K 8/60, A61Q 5/04, A61Q 5/06, A61Q 5/10, A61K 8/97, A61K 8/49

(54) **MITTEL MIT BIOFLAVONOID**
AGENTS CONTAINING BIOFLAVONOIDS
AGENTS CONTENANT UN BIOFLAVONOÏDE

(30) Priorität: 09.11.2007 DE 102007053950
(43) Veröffentlichungstag der Anmeldung: 14.07.2010
(73) Patentinhaber: Henkel AG & Co. KGaA, 40589 Düsseldorf (DE)
(72) Erfinder: KLEEN, Astrid, 20457 Hamburg (DE); RATH, Susanne, 20359 Hamburg (DE); AKRAM, Mustafa, 22455 Hamburg (DE)
(86) Internationale Anmeldenummer: PCT/EP2008/065031
(87) Internationale Veröffentlichungsnummer: WO 2009/060014

(56) Entgegenhaltungen:
- EP-A- 0 680 744
- WO-A-02/069926
- WO-A-2006/099930
- FR-A- 2 543 434
- JP-A- 2 138 114
- JP-A- 2001 270 812
- JP-A- 2003 048 818
- JP-A- 2005 145 882

## Beschreibung

Die vorliegende Anmeldung betrifft farbverandernde Mittel für keratinische Fasern, die mindestens Tillrosid enthalten sowie Haarbehandlungsverfahren mit derartigen Mitteln.

Menschliches Haar wird heute in vielfältiger Weise mit haarkosmetischen Zubereitungen behandelt Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe sowie der Veränderung der Form des Kopfhaares eine herausragende Rolle.

Die Veränderung der Form der Haare stellt einen wichtigen Bereich der modemen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden.

Gleichzeitig oder alternativ zu der Formveränderung besteht aber auch vielfach der Wunsch, die Haarfarbe zu ändern und zusammen mit der Haarfärbung auch eine Aufhellung des zu färbenden Haares zu erreichen.

Konventionelle Haarfärbemittel enthalten als farbverändemde Wirkstoffe in der Regel mindestens eine Entwickler- und/oder mindestens eine Kupplerkomponenten und gegebenenfalls noch direktziehende Farbstoffe als Nuanceure. Kuppler- und Entwicklerkomponenten werden auch als Oxidations-farbstoffvorprodukte bezeichnet. Die Grundlagen der Blondierverfahren sind dem Fachmann bekannt. Vor ihrer Anwendung auf menschliches Haar werden üblicherweise Haarfärbe- und/oder-aufhellungsmittel in fester oder pastöser Form mit einer verdünnten wässrigen Wasserstoffperoxid-Lösung vermischt. Die resultierende Mischung wird dann auf das Haar aufgebracht und nach einer bestimmten Einwirkzeit wieder ausgespült. Die Einwirkungsdauer auf dem Haar zur Erzielung einer vollständigen Ausfärbung beziehungsweise Aufhellung liegt in der Regel zwischen etwa 30 und 40 Minuten. Es ist nahe liegend, dass bei den Benutzern dieser Haarfarben oder Blondiermittel ein Bedürfnis besteht, diese Einwirkungszeit zu verringern.

Um eine ausreichenden Färbe- und/oder Blondierwirkung zu erzielen, sind derartige Mittel üblicherweise stark alkalisch eingestellt, der pH-Wert liegt dabei zwischen 8 und 10,5. Derart hohe pH-Werte sind erforderlich, um eine Öffnung der äußeren Schuppenschicht (Cuticula) zu gewährleisten und somit eine Penetration der aktiven Spezies (Wasserstoffperoxid) ins Haar zu ermöglichen.

Weder die pastenförmigen noch die pulverförmigen Färbe- und/oder Blondiermittel, die heute auf dem Markt sind, können als optimal angesehen werden. Während die Färbe- und/oder Blondierwirkung auf dem Haar als auf die Verbraucherbedürfnisse zugeschnitten bezeichnet werden kann, bestehen doch noch eine Reihe von Nachteilen und Problemen sowohl bei Herstellung als auch bei der Handhabung dieser Mittel.

Aufgrund der stark alkalischen Bedingungen sowie des Einsatzes von Wasserstoffperoxid kann es im Zusammenhang mit dem farbverändernden Vorgang zu Reizungen der Kopfhaut kommen. Diese sind sowohl während der Einwirkung des farbverändernden Mittels als auch Im Anschluss an die Behandlung zu beobachten.

Die dauerhafte Verformung von Keratinfasern wird üblicherweise so durchgeführt, dass man die Faser mechanisch verformt und die Verformung durch geeignete Hilfsmittel festlegt Vor und/oder nach dieser Verformung behandelt man die Faser mit der wässrigen Zubereitung einer keratinreduzierenden Substanz und spült nach einer Einwirkungszeit mit Wasser oder einer wässrigen Lösung. In einem zweiten Schritt behandelt man dann die Faser mit der wässrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wässrige Zubereitung der keratinreduzierenden Substanz ist üblicherweise alkalisch eingestellt, damit zum einen genügender Anteil der Thiolfunktionen deprotoniert vorliegt und zum anderen die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so dass es zu einer Lockerung der Peptidvernetzung und infolge der Spannung der Faser durch die mechanische Verformung zu einer Neuorientierung des Keratingefüges kommt. Unter dem Einfluss des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Auch bei der Dauerwellbehandlung werden die Fasern aggressiven Medien ausgesetzt, so dass auch In diesem Bereich ein Bedürfnis bestand, die Mittel hinsichtlich ihrer pflegenden Eigenschaften weiter zu verbessern.

Daher hat es in der Vergangenheit nicht an Versuchen gefehlt, die farb- und/oder form verändernden Mittel sowohl hinsichtlich ihrer Kopfhaut- als auch ihrer Haarverträglichkeit zu optimieren. Dabei wurden sowohl Methoden vorgeschlagen, bei denen die Kopfhaut und/oder die Haare vorab mit einer schützenden Zubereitung vorbehandelt wurde, als auch Methoden, bei denen die farb- und/oder formverändernden Mittel selber Wirkstoffe enthalten, die die Kopfhautirritationen minimieren sowie die Haarschädigungen vermeiden sollen. Dennoch konnten die Mittel des Standes der Technik die bestehenden Anforderungen bisher nicht vollstandig erfüllen.

Die im Rahmen dieser Methoden einsetzbaren Wirkstoffe müssen Insbesondere hinsichtlich ihrer Stabilität hohen Ansprüchen genügen, da die Färbecremes und Wellmittel üblicherweise einen hohen pH-Wert und die Oxidationsmittelzubereitungen einen niedrigen pH-Wert aufweisen. Auch müssen diese Wirkstoffe gegenüber den reduzierenden Bedingungen der Wellmittel stabild sein. Ferner sind Unverträglichkeiten der verschiedenen Wirkstoffe untereinander und somit eine geringe Lagerstabilität zu vermeiden.

Es bestand daher weiterhin Bedarf an Wirkstoffen, die unter den Bedingungen der oxidativen Farbveränderung sowie der Dauerwellbehandlung keratinischer Fasern die Irritationen der Kopfhaut, die sich in Form von Kribbeln, Jucken und/oder Rötungen äußern können, herabsetzen können und zusätzlich antimikrobiell, entzündungshemmend und/oder antioxidierend wirken können. Ferner besteht ein Interesse an Wirkstoffen, die weiterhin besonders gut die primären Ziele der erfindungsgemäßen Mittel unterstützen und zu intensiveren Färbungen, verbesserten Echtheitseigenschaften oder zu stabileren Dauerwellen führen. Weiterhin sollten diese Wirkstoff auch einen positiven Einfluß auf den Pflegezustand der Haare ausüben.

Die Dokumente FR 2543434 A1 und JP 2001/270812 A beschreiben Haarfärbemittel, welche Rutin enthalten. JP 2005/145882 A offenbart einen aus zwei Komponenten bestehenden Haarfärbekit, wobei die erste Komponente Farbstoffvorprodukte und die zweite Komponente Wassserstoffperoxid und Rutinosid enthält. In JP 2003/048818 A werden Haarfärbemittel mit Henna und Rutin beansprucht. Die in JP 2138114 A offenbarten Haarfärbemittel enthalten Rutin und Quercetin. EP 0680744 A1 beschreibt die Verwendung von Bioflavonoiden wie beipielsweise Rutin zur Verbesserung der mechanischen Eigenschaften der Haare.WO 02/069926 A1 betrifft kosmetische Formulierungen mit Bioflavonoid-Derivaten. In WO 2006/099930 A1 werden Pflanzenextrakte beschrieben, die u.a. Tilirosid enthalten können.

Es wurde nunmehr überraschenderweise gefunden, dass farbverändernde Mittel, die neben dem Wirkstoff, der die Farbe der keratinischen Fasern verändert, mindestens ein spezielles Flavonoid enthalten, stabil sind, hinsichtlich ihrer Farb- und/oder Formveränderung zufrieden stellen und gleichzeitig eine geringere Kopfhautirritation verursachen. Darüber hinaus weisen die mit den erfindungsgemäßen Mitteln behandelten Fasern einen besseren Pflegezustand und eine stabilisierte Struktur auf.

Ein erster Gegenstand der Erfindung ist ein Mittel zur Farbveränderung keratinischer Fasern, enthaltend in einem kosmetisch akzeptablen Träger mindestens
- mindesten ein Oxidationsfarbstoffvorprodukt vom Entwickler- und/oder Kuppler-Typ;
- Tilirosid.

Unter "keratinischen Fasern" sind dabei erfindungsgemäß Pelze, Wolle, Federn und insbesondere menschliche Haare zu verstehen.

### Erfindungsgemäß ist

- Tilirosid ([(2R,3R,4S,5R,6S)-6-[5,7-dihydroxy-2-(4-hydroxyphenyl)-4-oxo-chromen-3 -yl]oxy-3,4,5-trihydroxy-oxan-2-yl]methyl (E)-3-(4-hydroxyphenyl)prop-2-enoate; INCI-Bezeichnung: Tiliroside, CAS-Nr. 20316-62-5).

In der WO 02/69926 wird angegeben, dass Tilirosid beispielsweise aus den Pflanzen der Gattung Althaea, Aristolochia, Helianthemum, Lindera, Magnolia, Platanus, Potentilla, Quercus, Rosa, Sida, Sorbus und/oder Tilia gewonnen werden kann, wobei folgende Arten bevorzugt sind: Althaea officinalis, Althaea rosea, Aristolochia heterophylla, Helianthemum glomeratum, Lindera megaphyfla, Magnolia salicifolia, Platanus acerifolia, Platanus occidentalis, Potentilla anserina, Quercus pubescens, Quercus suber, Quercus laurifolia, Quercus ilex, Quercus imbricaria, Quercus virginiana, Rosa pomifera, Sida rhombifolia, Sida poeppigiana, Sida cordifolia, Sida glaziovii, Sorbus pendula, Tilia argenta und Tilia cordata.

In der WO 06/099930 wird weiterhin beschrieben, dass Tilirosid aus Pflanzen der Familie der Sterculiaceae gewonnen werden kann. Als bevorzugt werden dort die Waitheria Spezies, vorzugsweise Waitheria americana, Waitheria douradinha, Waitheria panicucata, Waitheria indica, Waitheria viscosissima, Waitheria antennalls, Waitheria ovata, Waitheria tornentosa, Waitheria madagascariensis, Waitheria glornerata, Waitheria bicolor, Waitheria fryxellii, Waitheria tundeltiana, Waltheria tridentata, Waitheria operculata, Waitheria bracteosa, Waitheria douradinha, Waitheria macropoda, Waitheria caroliniana, Waitheria arenicola, Waitheria melochia, Waitheria acurninata, Waitheria theobroma, Waitheria indivia oder Waitheria taiwana genannt.

Entsprechende Verfahren zur Gewinnung des Rohstoffs sind in den beiden genannten Schriften offenbart.

Tilirosid wird als kosmetischer Wirkstoff beispielsweise unter der Handelsbezeichnung Ronacare^{®} Tiliroside von der Fa. Merck kommerziell vertrieben. Es handelt sich dabei um eine Mischung von ca. 5 Gew.-% reines Tilirosid mit ca. 95 Gew.% Sorbitol.

Auch wenn die Abmischung mit Sorbitol erfindungsgemäß bevorzugt ist, ist auch ein Einsatz von reinem Tilirosid oder in Kombination mit anderen pulverförmigen Stellmitteln erfindungsgemäß möglich.

Das erfindungsgemäß Flavonoide Ist in den Farbemitteln vorzugsweise in einer Menge von 0,001 bis 5Gew.-%, jeweils bezogen auf den reinen Wirkstoff und das gesamte anwendungsbereite Mittel, enthalten.

Als weiteren erfindungswesentlichen Bestandteil enthalten die Färbemittel mindestens ein Oxidationsfarbstoffvorprodukt vom Entwickler und/oder Kuppler-Typ.

Die erfindungsgemäßen Färbemittel enthalten als Farbstoffvorprodukte
- Oxidationsfarbstoffvorprodukte vom Entwickler- und/oder Kuppler-Typ, und gegebenenfalls zusätzlich
- Vorstufen naturanaloger Farbstoffe, wie Indol- und Indolin-Derivate,

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-' phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Kupplerkomponenten bilden Im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3aminophenol, 3-Trifluoroacetylamino-2-chlor-6-methylphenol, 5-Amino-4-chlor-2-methylphenyl, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methy]phenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 6-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol. Weiterhin können die Mittel zusätzlich zu den Farbstoffvorprodukten mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehenden Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternaren Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

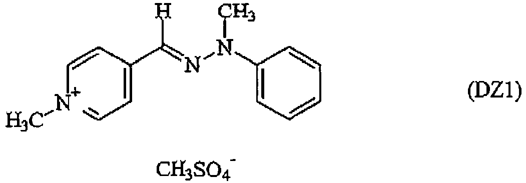

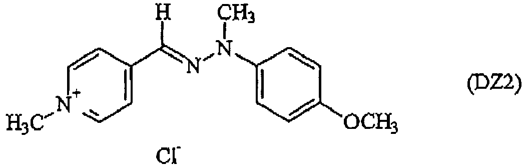

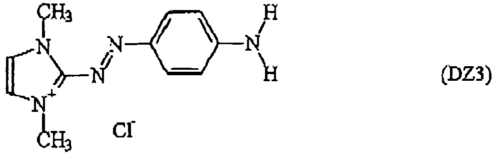

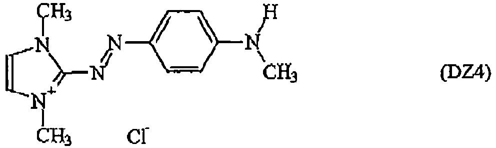

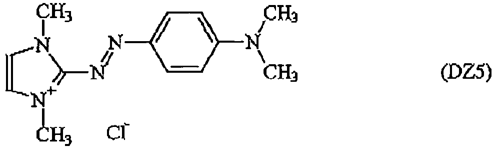

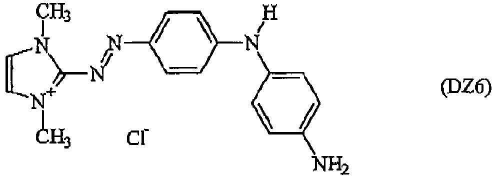

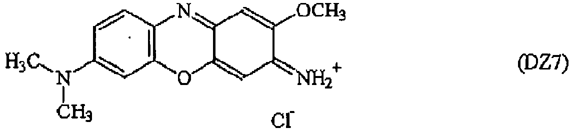

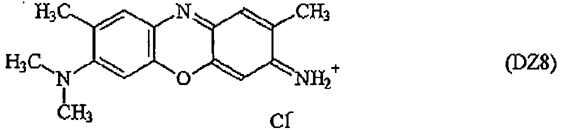

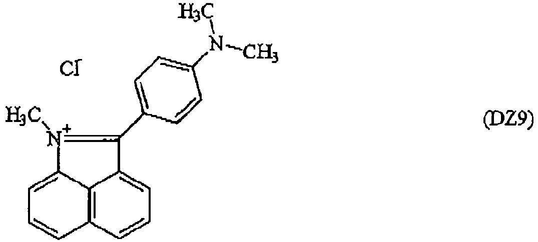

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel enthalten die direktziehenden Farbstoffe bevorzugt in einer Menge von 0,01 bis 20 Gew.-%, bezogen auf die gesamte Anwendungszubereitung.

Es hat sich gezeigt, das erfindungsgemäßauch Zwei- und Mehrphasensysteme bevorzugt sind. Dies sind Systeme, bei denen mindestens zwei separate, kontinuierliche Phasen vorliegen. Beispiele für solche Systeme sind Zubereitungen, die folgende Phasen aufweisen:
- eine wässrige Phase und eine nichtwässrige Phase, die separat voneinander vorliegen
- eine wässrige Phase und zwei nichtwässrige, miteinander nicht mischbare Phasen, die jeweils separat vorliegen
- eine Öl-in-Wasser-Emulsion und eine davon separiert vorliegende nichtwässrige Phase
- eine Wasser-in-Öl-Emulsion und eine davon separiert vorliegende wässrige Phase.

Es hat sich erfindungsgemäß als vorteilhaft erwiesen, wenn die Mittel der vorliegenden Erfindung neben dem speziellen Flavonoid einen weiteren Wirkstoff enthalten, der pflegende, entzündungshemmende, anti-irritierende und/oder hautberuhigende Eigenschaften aufweist.

Im Rahmen einer ersten bevorzugten Ausführungsform enthält das erfindungsgemäße farbverändernde Mittel als Pflegestoff mindestens ein kationisches Tensid.

Die kationischen Tenside sind in den erfindungsgemäß verwendeten Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Im Rahmen einer zweiten bevorzugten Ausführungsform der vorliegenden Erfindung enthalten die farbverändernden Mittel als Pflegestoff mindestens ein pflegendes Polymer.

Die erfindungsgemäßen farbverändernden Mittel enthalten die kationischen Polymere bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Im Rahmen einer dritten bevorzugten Ausführungsform enthalten die erfindungsgemäßen farbverändernden Mittel mindestens einen UV-Filter. Die erfindungsgemäßgeeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestem, Salicylsäureestern, Benzimidazolen und o-Aminobenzoesäureestem.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyldimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt Die UV-Filter sind in den erfindungsgemäßen Mitteln üblicherweise in Mengen 0,01-5 Gew.%, bezogen auf die gesamte Anwendungszubereitung, enthalten. Mengen von 0,1-2,5 Gew.-% sind bevorzugt.

Im Rahmen einer vierten bevorzugten Ausführungsform enthalten die erfindungsgemäßen farbverändernden Mittel als Pflegestoff mindestens ein Vitamin, ein Provitamin, eine Vitaminvorstufe sowie eines derer Derivate.

Dabei sind erfindungsgemäß solche Vitamine, Pro-Vitamine und Vitaminvorstufen bevorzugt, die üblicherweise den Gruppen A, B, C, E, F und H zugeordnet werden.

Vitamin H. Als Vitamin H wird die Verbindung (3aS,4S, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.%, jeweils bezogen auf die gesamte Anwendungszubereitung enthalten.

Bevorzugt enthalten die erfindungsgemäßen farbverändernden Mittel Vitamine, Provitamine und Vitaminvorstufen aus den Gruppen A, B, C, E und H. Das Vitamin C kann erfindungsgemäß ganz besonders bevorzugt sein.

Panthenol, Pantolacton, Pyridoxin und seine Derivate sowie Nicotinsäureamid und Biotin sind besonders bevorzugt.

Im Rahmen einer fünften bevorzugten Ausführungsform enthalten die erfindungsgemäßen farbverändemden Mittel mindestens einen Pflanzenextrakt.

Üblicherweise werden diese Extrakte durch Extraktion der gesamten Pflanze hergestellt Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Besonders bevorzugt sind die Extrakte aus Moringa Olifeira, Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Moringa Olifeira, Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen farbverändernden Mitteln Mischungen aus mehreren, insbesondere aus zwei, verschiedenen Pflanzenextrakten einzusetzen.

Im Rahmen einer sechsten Ausführungsform enthalten die erfindungsgemäßen farbverändernden Mittel als Pflegestoff mindestens eine Carbonsäure.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei Ci- bis C₄-Alkylgruppen trägt. Das Natriumsalz ist ganz besonders bevorzugt. Die eingesetzten Mengen in den erfindungsgemaßen Mitteln betragen 0,05 bis 10 Gew.%, bezogen auf die gesamte Anwendungszubereitung, besonders bevorzugt 0,1 bis 5, und insbesondere 0,1 bis 3 Gew.%.

Bevorzugte Hydroxycarbonsäureester sind beispielsweise Vollester der Glycolsäure, Milchsäure, Äpfelsäure, Weinsäure oder Citronensäure. Weitere grundsätzlich geelgneten Hydroxycarbonsäureester sind Ester der ß-Hydroxypropionsäure, der Tartronsaure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Im Rahmen einer siebten bevorzugten Ausführungsform enthalten die erfindungsgemäßen farbverändemden Mittel als Pflegestoff mindestens ein Proteinhydrolysat und eines seiner Derivate. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfingdungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich. Erfindungsgemäß ist es auch möglich, eine Mischung aus mehreren Proteinhydrolysaten einzusetzen. Die Proteinhydrolysate sind in den erfindungsgemäßen farbverändernden Mitteln in Konzentrationen von 0,01 Gew. -% bis zu 20 Gew.%, vorzugsweise von 0,05 Gew.% bis zu 15 Gew.-% und ganz besonders bevorzugt in Mengen von 0,05 Gew.-% bis zu 5 Gew.%, jeweils bezogen auf die gesamte Anwendungszubereitung enthalten.

Im Rahmen einer achten bevorzugten Ausführungsform enthalten die erfindungsgemäßen Zubereitungen als Pflegestoff Ectoin oder Ectoinderivate, Allantoin, Taurin und Bisabolol enthalten.

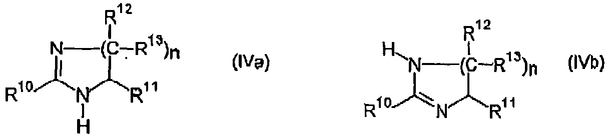

Erfingdungsgemäß werden unter dem Begriff "Ectoin und Ectoinderivate" Verbindungen der Formel (IV) und/oder deren physiologisch verträglichen Salzes und/oder einer isomeren oder stereoisomeren Form verstanden, wobei
- R¹⁰ steht für ein Wasserstoffatom, einen verzweigten oder unverzweigten C₁ - C₄-Alkylrest oder einen C₂ - C₄-Hydroxyalkylrest,
- R¹¹ steht für ein Wasserstoffatom, eine Gruppierung -COOR¹⁴ oder eine Gruppierung-CO(NH)R¹⁴, wobei R¹⁴ für ein Wasserstoffatom, einen C₁ - C₄-Alkylrest, einen Aminosäurerest, einen Dipeptid- oder einen Tripeptidrest stehen kann,
- R¹² und R¹³ stehen unabhängig voneinander für ein Wasserstoffatom, einen C₁ - C₄-Alkylrest oder einer der beiden Reste steht für eine Hydroxygruppe und
- n steht für eine ganze Zahl von 1 bis 3.

Geeignete physiologisch verträgliche Salze der allgemeinen Verbindungen gemäß der Formel (IVa) oder (IVb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IVa) oder (IVb) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) C₁ - C₄- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IVa) oder (IVb), sowie die Salze, die sich durch Umsetzung von Verbindungen gemäß der Formel (IVa) oder (IVb) mit Salzsäure, Essigsäure, Citronensäure und Benzoesäure ergeben.

Unter isomeren oder stereoisomeren Formen der Verbindungen gemäß Formel (IVa) oder (IVb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden:
Asparagin, Arginin, Asparaginsäure, Glutamin, Glutaminsäure, β-Alanin, γ-Aminobutyrat, N_{ε}-Acetyllysin, N_{δ}-Acetylomitin, N_{γ}-Acetyldiaminobutyrat, N_{α}-Acetyldiaminobutyrat, Histidin, Isoleucin, Leucin, Methionin, Phenylalanin, Serin, Threonin und Tyrosin

L-Aminosäuren sind bevorzugt. Aminosäurereste leiten sich von den entsprechenden Aminosäuren ab. Die folgenden Aminosäurereste sind bevorzugt:
Gly, Ala, Ser, Thr, Val, β-Ala, γ-Aminobutyrat, Asp, Glu, Asn, Aln, N_{ε}-Acetyllysin, N_{δ}-Acetylomithin, N_{γ}-Acetyldiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für C₁ - C₄-Alkylgruppen in den erfindungsgemäßen Verbindungen sind Methyl, Ethyl, n-Propyl, Isopropyl, n-Butyl, Isobutyl und tert-Butyl. Bevorzugte Alkylgruppen sind Methyl und Ethyl, Methyl ist eine besonders bevorzugte Alkylgruppe. Bevorzugte C₂ - C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Die erfindungsgemäßen farbverändemden Mittel enthalten diese Wirkstoffe bevorzugt in Mengen von 0,001 bis 2, insbesondere von 0,01 bis 0,5 Gew.-%, jeweils bezogen auf die gesamte Anwendungszubereitung.

Im Rahmen einer neunten bevorzugten Ausführungsform enthalten die erfindungsgemäßen farbverändernden Mittel als Pflegestoff mindestens ein Mono- bzw. Oligosaccharid.

Es können sowohl Monosaccharide als auch Oligosaccharide, wie beispielweise Rohrzucker, Milchzucker und Raffinose, eingesetzt werden. Die Verwendung von Monosacchariden ist erfindungsgemäß bevorzugt Unter den Monosacchariden sind wiederum solche Verbindungen bevorzugt, die 5 oder 6 Kohlenstoffatome enthalten.

Weiterhin hat es sich als vorteilhaft erwiesen, wenn die erfindungsgemaßen Zubereitungen eine erhitzte, das heißt karamellisierte ZucKerart, vorzugsweise karamellisierten Rohrzucker enthalten.

Die Mono- bzw. Oligosaccharide sind in den erfindungsgemäßen Haarbehandlungsmitteln bevorzugt in einer Menge von 0,1 bis 8 Gew.-%, insbesondere 1 bis 5 Gew.-%, bezogen auf die gesamte Anwendungszubereitung, enthalten.

Im Rahmen einer zehnten Ausführungsform enthält die erfindungsgemäße Zubereitung (B) als Pflegestoff mindestens ein Silikonöl und/oder ein Silikongum.

Erfindungsgemäß geeignete Silikone oder Silikongums sind insbesondere Dialkyl- und Alkylarylsiloxane, wie beispielsweise Dimethylpolysiloxan und Methylphenyl-polysiloxan, sowie deren alkoxylierte, quaternierte oder auch anionische Derivate.

Gemäß einer bevorzugten Ausführungsform enthalten die erfindungsgemäßen Zubereitungen eine Kombination aus einem flüchtigen und einem nichtflüchtigen Silikon. Flüchtig im Sinne der Erfindung sind solche Silikone, die ein Flüchtigkeit aufweisen, die gleich oder größer als die Flüchtigkeit des cyclischen, pentameren Dimethylsiloxans ist. Solche Kombinationen sind auch als Handelsprodukte (z. B. Dow Coming^{®}1401, Dow Corning^{®}1403 und Dow Corning^{®}1501, jeweils Mischungen aus einem Cyclomethicone und einem Dimethiconol) erhältlich.

Gemäß einer besonders bevorzugten Ausführungsform wird als Komponente (A) ein Dialkylpolysiloxan oder eines seiner Derivate eingesetzt Bevorzugt sind die Alkylgruppen Methyl, Ethyl, i-Propyl und n-Propyl. Dimethylpolysiloxan oder eines seiner Derivate wird besonders bevorzugt eingesetzt. Bevorzugte sind die Derivate des Dimethylplysiloxans, die aminofunktionell sind. Ein ganz besonders bevorzugtes Derivat ist unter der INCI-Bezeichnung Amodimethicone im Handel erhältlich.

Die erfindungsgemäßen Zubereitungen enthalten die Silikone bevorzugt in Mengen von 0,01 - 10 Gew.-%, Insbesondere 0,1 - 5 Gew.-%, bezogen auf die gesamte Anwendungszubereitung.

Im Rahmen einer elften Ausführungsform enthalten die erfindungsgemäßen farbverändernden Mittel mindestens ein Lipid als Pflegestoff.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Die erfindungsgemäßen Zubereitungen enthalten die Lipide bevorzugt in Mengen von 0,01 - 10 Gew.-%, insbesondere 0,1 - 5 Gew.-%, bezogen auf die gesamte Anwendungszubereitung.

Im Rahmen einer zwölften Ausführungsform enthalten die erfindungsgemäßen farbverändernden Mittel mindestens ein Ölkörper und/oder einen Wachs als Pflegestoff.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle,
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Din-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie di-tert-butylether, Di-iso-pentylether, Di-3-ethyldecylether, tert-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfetta[koho]-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-dl(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetil^{®} CC),
- Trifettsäureester von gesättigten und/oder ungesättigten linearen und/oder verzweigten Fettsäuren mit Glycerin, Fettsäurepartialglyceride, das sind Monoglyceride, Diglyceride und deren technische Gemische. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensaure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen farbverändernden Mitteln beträgt üblicherweise 0,1 -30 Gew.%, bezogen auf die gesamte Anwendungszubereitung, bevorzugt 0,1 - 20 Gew.-%, und insbesondere 0,1 -15 Gew.-%.

Weiterhin hat es sich als vorteilhaft erwiesen, wenn die erfindungsgemäßen Mittel mindestens eine Esterverbindung enthalten, die als Alkoholkomponente einen gesättigten oder ungesättigten, verzweigten oder unverzweigten Wachsalkohol enthält.

Dabei haben sich Insbesonder die gesättigten Wachsalkohole als bevorzugt erwiesen. Weiterhin haben sich die unverzweigten Wachsalkohole als bevorzugt erwiesen. Besonders bevorzugt sind die gesättigten und unverzweigten Wachsalkohole. Ebenfalls bevorzugt sind Wachsalkohole mit einer endständigen Hydroxygruppe. Beispiele für erfindungsgemäß bevorzugte Wachsalkohole sind Tetracosan-1-ol, Hexacosan-1-ol, Triacontan-1-ol sowie Hentriacontan-1-ol. Triacontan-1-ol ist erfindungsgemäß ganz besonders bevorzugt.

Als weiteren Bestandteil enthält die erfindungsgemäße Esterverbindung mindestens eine Fettsäurekomponente. Die Fettsäurekomponente weist vorzugsweise 10 bis 30 Kohlenstoffatome, insbesondere 16 bis 26 Kohlenstoffatome auf. Dabei sind sowohl gesättigte als auch ungesättigte sowie verzweigte und unverzweigte Fettsäuren prinzipiell geeignet Als bevorzugte Fettsäuren kommen insbesondere die unverzweigten gesättigten Fettsäuren, wie beispielsweise Tetradecansäure, Pentadecansäure, Palmitinsäure, Margarinsäure, Stearinsäure, Nonadecansäure, Arachinsäure, Behensäure, Tetracosansäure, Cerotinsäure sowie Triacontansäure in Frage. Palmitinsäure, Stearinsäure, Behensäure und Triacontansäure sind erfindungsgemäß besonders bevorzugt

Besonders vorteilhaft im Sinne der vorliegenden Erfindung ist es ferner, wenn mindestens zwei verschiedene Esterverbindungen in den erfindungsgemäßen Mitteln enthalten sind, wobei die erste Esterverbindung eine Fettsäurekomponente mit 12 bis 18 Kohlenstoffatomen und die zweite Esterverbindung eine Fettsäurekomponente mit 22 bis 30 Kohlenstoffatomen aufweist.

Ein Beispiel für eine derartige Kombination sind Esterverbindungen auf Basis von Palmitinsäure und Cerotinsäure.

Ganz besonders bevorzugte Esterverbindungen sind die Ester von Triacontan-1-ol mit Palmitinsäure und Cerotinsäure.Derartige Esterverbindungen oder auch deren Mischungen können beispielsweise aus natürlichern Bienenwachs gewonnen werden.

Es hat sich aber erfindungsgemäß auch als vorteilhaft erwiesen, wenn das komplexe Gemisch des natürlichen Bienenwachses, das eine erfindungsgemäße Esterverbindung umfasst, als solches in den erfindungsgemäßen Mitteln zum Einsatz kommt.

Als natürliche oder synthetische Wachse können ferner erfindungsgemäß eingesetzt werden feste Paraffine oder Isoparaffine, Carnaubawachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Wirkstoffkombination, die aus den erfindungsgemäßen Flavonoiden und Bienenwachs gebildet wird, ist erfindungsgemäß besonders bevorzugt, da durch ihren Einsatz der Haaroberfläche und das Haarinnere überraschenderweise vermehrt gepflegt werden kann.

Im Rahmen einer dreizehnten Ausführungsform enthalten die erfindungsgemäßen Zubereitungenein Enzym als Pflegestoff. Erfindungsgemäß besonders bevorzugte Enzyme sind ausgewählt aus einer Gruppe, die gebildet wird aus Proteasen, Lipasen, Transglutaminase, Oxidasen und Peroxidasen. Obwohl jeder der in den verschiedenen Ausführungsformen genannten Pflegestoffe für sich alleine bereits ein zufriedenstellendes Resultat ergibt, sind im Rahmen der vorliegenden Erfindung auch alle Ausführungsformen umfasst, in denen die farbverändernden Mittel mehrere Pflegestoffe auch aus verschiedenen Gruppen enthalten.

Besonders bevorzugte Pflegestoffe im Sinne der vorliegenden Erfindung sind insbesondere Vitamin C, Ubiquinone, Panthenol und seine Derivate, Ectoin, Kamillenextrakt, Bisabolol, Olivenöl, Royal Gelee, Mung Bean Extrakt und Moringa Olifeira-Extrakt.

Die erfindungsgemäßen farbverandernden Mittel können neben den erfindungswesentlichen Komponenten weiterhin alle für solche Zubereitungen bekannten Wirk-, Zusatz- und Hilfsstoffe enthalten. In vielen Fällen enthalten die farbverändernden Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Hinsichtlich der kationischen Tenside sei an dieser Stelle auf die obigen Ausführungen verwiesen.

Ferner können die erfindungsgemäßen farbverändernden Mittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-tri-methylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere. Vinylaretat/Butylmaleat/Isobornylacrylat-Copolymere, Methylvinylether/Malein-saureanhydrid-Copolymere und Acrylsäure/Ethylacrylat/N-tertButyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellu-lose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternislerte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten.

Die farbverändernden Mittel enthalten die erfindungswesentlichen Komponenten erfindungsgemäß bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Weiterhin können die erfindungsgemäßen farbverändernden Mittel ein Reduktionsmittel enthalten. Beispiele für erfingdungsgemäß bevorzugte Reduktionsmittel sind Natriumsulfit, Ascorbinsaure, Thioglykolsäure und deren Derivate, Natriumthionit, Alkalimetallcitratsalze und N-Acetyl-L-Cystein Ganz besonders bevorzugte Reduktionsmittel sind Alkalimetallcitratsalze, insbesondere Natriumcitrat, und N-Acetyl-L-Cystein. N-Acetyl-L-Cystein ist ein ganz besonders bevorzugtes Reduktionsmittel.

Weiterhin können die erfindungsgemäßen Mittel Alkalisierungsmittel, üblicherweise Alkali- oder Erdal-kalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Häufig werden in Färbemittel Perlglanzpigmente eingesetzt Erfindungsgemäß bevorzugte Perlglanzpigmente sind natürliche Perlglanzpigmente wie z. B. Fischsilber (Guanin/Hypoxanthin-Mischkristalle aus Fischschuppen) oder Perlmutt (aus vermahlenen Muschelschalen), monokristalline Perlglanzpigmente wie z.B. Bismutoxychlorid, sowie Perlglanzpigmente auf Basis von Glimmer oder Glimmer/Metalloxid, Durch den Einsatz der Perlglanzpigmente werden Glanz und gegebenenfalls zusätzlich Farbeffekte in den erfindungsgemäßen Mitteln erzielt Die Farbgebung durch die in den Mitteln verwendeten Perlglanzpigmente beeinflusst das Farbergebnis der Färbung der Keratinfasern jedoch nicht

Perlglanzpigmente auf Glimmer-Basis und auf Glimmer/Metalloxid-Basis sind erfindungsgemäß ebenfalls bevorzugt. Die wichtigsten Vertreter dieser Silicate sind Muscovit, Phlogopit, Paragonit, Blotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. TiO₂, Cr₂O₃ und Fe₂O₃. Durch entsprechende Beschichtung werden Interferenzpigmente sowie Farbglanzpigmente als erfindungsgemäße Perlglanzpigmente erhalten. Diese Perlglanzpigmentarten weisen neben einem glitzernden optischen Effekt zusätzlich Farbeffekte auf. Desweiteren können die erfindungsgemäß verwendbaren Perlglanzpigmente weiterhin ein Farbpigment enthalten, welches sich nicht von einem Metalloxid ableitet

Die Korngröße der bevorzugt verwendeten Perlglanzpigmente liegt bevorzugt zwischen 1.0 und 100 µm, besonders bevorzugt zwischen 5.0 und 60.0 µm.

Besonders bevorzugte Perlglanzpigmente sind Pigmente, die von der Firma Merck unter den Handelsnamen Colorona^{®} vermarktet werden, wobei die Pigmente Colorona^{®} red-brown (47-57 Gew.% Muscovit Mica (KH₂(AlSiO₄)₃), 43-50 Gew.% Fe₂O₃ (INCI: Iron Oxides CI 77491), <3 Gew.% TiO₂ (INCI: Titanium Dioxide CI 77891), Colorona^{®} Blackstar Blue (39-47 Gew.% Muscovit Mica (KH₂(AlSiO₄)₃), 53-61 Gew.% Fe₃O₄ (INCI: Iron Oxides CI 77499)), Colorona^{®} Siena Fine (35-45 Gew.% Muscovit Mica (KH₂(AlSiO₄)₃), 55-65 Gew.% Fe₂O₃ (INCI: Iron Oxides CI 77491)), Colorona^{®} Aborigine Amber (50-62 Gew.% Muscovit Mica (KH₂(AlSiO₄)₃), 36-44 Gew.% Fe₃O₄ (INCI: Iron Oxides CI 77499), 2-6 Gew.% TiO₂ (INCI: Titanium Dioxide CI 77891)), Colorona^{®} Patagonian Purple (42-54 Gew.% Muscovit Mica (KH₂(AlSiO₄)₃), 26-32 Gew.% Fe₂O₃ (INCI: Iron Oxides CI 77491), 18-22 Gew.% TiO₂ (INCI: Titanium Dioxide CI 77891), 2-4 Gew.% Preussisch Blau (INCI: Ferric Ferrocyanide CI 77510)), Colorona^{®} Chameleon (40-50 Gew.% Muscovit Mica (KH₂(AlSiO₄)₃), 50-60 Gew.% Fe₂O₃ (INCI: Iron Oxides Cl 77491)) und silk^{®} Mica ( >98 Gew.% Muscovit Mica (KH₂(AlSiO₄)₃)).

Erfolgt die Ausbildung der eigentlichen Färbung im Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Bevorzugt wird jedoch ein chemisches Oxidationsmittel eingesetzt, besonders dann, wenn neben der Färbung ein Aufhelleffekt an menschlichem Haar gewünscht ist Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbernittel auch zusammen mit einem Katalysator auf das Haar aufgebracht werden, der die Oxidation der Farbstoffvorprodukte, z.B. durch Luftsauerstoff, aktiviert Solche Katalysatoren sind z.B. Metallionen, Iodide, Chinone oder bestimmte Enzyme.

Das eigentliche oxidative Färbemittel wird zweckmäßigerweise unmittelbar vor der Anwendung durch Mischung der Zubereitung des Oxidationsmittels mit der Zubereitung, enthaltend die Farbstoffvorprodukte, hergestellt Das dabei entstehende gebrauchsfertige Haarfärbepräparat sollte bevorzugt einen pH-Wert im Bereich von 5 bis 14, insbesondere von 7 bis 12, aufweisen. Besonders bevorzugt ist die Anwendung der Haarfärbemittel in einem schwach alkalischen Milieu. Die Anwendungstemperaturen können In einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde. Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampoonlert. Bei dieser Ausführungsform wird gemäß einer ersten Variante, bei der das vorherige Aufbringen der Farbstoffvorprodukte eine bessere Penetration In das Haar bewirken soll, das entsprechende Mittel auf einen pH-Wert von etwa 4 bis 7 eingesteltt Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Erfindung ist ein Verfahren zur Farbveränderung keratinischer Fasern, bei dem ein erfindungsgemäßes Mittel mit einer Oxidationsmittelzubereitung vermischt wird, die Anwendungszubereitung auf die Fasern aufgetragen wird und nach einer Einwirkzeit wieder abgespült wird.

Ein dritter Gegenstand der vorliegenden Erfindung Ist ein Verfahren zur Farbveränderung keratinischer Fasern, bei dem in einem ersten Schritt ein Mittel, enthaltend mindestens ein Flavonoid der Formel (I), auf die Fasern aufgetragen wird, nach einer Einwirkzeit ein zweites Mittel, enthaltend mindestens einen farbverändernden Wirkstoff, auf die Fasern aufgetragen wird und in einem dritten Schritt die Mittel nach einer weiteren Einwirkzeit abgespült werden.

Dabei hat es sich als besonders vorteilhaft erwiesen, wenn die erste Einwirkzeit von 1 bis 60 Minuten, vorzugsweise 1 bis 15 Minuten, inbesondere 1 bis 5 Minuten und die zweite Einwirkzeit von 1 bis 60 Minuten, vorzugsweise 30 bis 45 Minuten betragen.

Ein vierter Gegenstand der vorliegenden Erfindung ist Verfahren zur Farbveränderung keratinischer Fasern, bei dem in einem ersten Schritt ein Mittel, enthaltend mindestens einen farbverändernden Wirkstoff, auf die Fasern aufgetragen wird, nach einer Einwirkzeit ein zweites Mittel, enthaltend mindestens ein Flavonoid der Formel (I), auf die Fasern aufgetragen wird und in einem dritten Schritt die Mittel nach einer weiteren Einwirkzeit abgespült werden.

Im Rahmen des vierten Gegenstandes der vorliegenden Erfindung hat es sich als besonders vorteilhaft erwiesen, wenn die erste Einwirkzeit von 1 bis 60 Minuten, vorzugsweise 1 bis 15 Minuten, inbesondere 1 bis 5 Minuten, und die zweite Einwirkzeit von 1 bis 60 Minuten, vorzugsweise 30 bis 45 Minuten, betragen.

Bezüglich bevorzugter Einsatzkonzentrationen, bevorzugter weiterer Inhaltsstoffe der jeweiligen verwendeten Mittel und weitere bevorzugter Ausführungsformen des zweiten bis vierten Gegenstandes gilt mutatis mutandis das zu den erfindungsgemäßen Mitteln Gesagte.

Die nachfolgenden Beispiele sollen den Gegenstand der vorliegenden Anmeldung verdeutlichen ohne ihn In irgendeiner Welse zu beschränken.

### Beispiele

Die Mengenangaben verstehen sich, soweit nichts anderes vermerkt ist, jeweils in Gewichtsprozent.

### 1 Herstellung der Zubereitungen

### 1.1 Herstetlung der Farbrezepturen

| Rohstoff | Farbrezeptur A | Farbrezeptur B | Farbrezeptur C | Farbrezeptur D |
|---|---|---|---|---|
| Xanthan^{®} FN | 0,1 | 0,1 | 0,1 | 0,1 |
| Ectoin | 0,1 | 0,5 | 1,2 | 2,0 |
| Ronacare^{®} Tiliroside | 0,01 | 2,0 | 0,5 | 0,05 |
| Cetiol^{®} V | 2,3 | 2,3 | 2,3 | 2,3 |
| Lanette^{®} N | 14,0 | 14,0 | 14,0 | 14,0 |
| Cetearyl Alcohol | 3,9 | 3,9 | 3,9 | 3,9 |
| Cutina^{®} GMS SE | 6,0 | 6,0 | 6,0 | 6,0 |
| Kokosamidopropylbetain 40 % | 2,0 | 2,0 | 2,0 | 2,0 |
| Monoethanolamin | 0,2 | 0,4 | 0,3 | 0,3 |
| Karamelzuckersirup 75 % | 0,1 | 0,1 | 0,1 | 0,1 |
| Ascorbinsäure | 0,1 | 0,1 | 0,1 | 0,1 |
| Natriumsulfit | - | 0,3 | - | 0,2 |
| Monoethanolamin | 3,0 | 3,0 | 3,0 | 3,0 |
| p- Toluylendiaminsulfat | 0,7 | 1,5 | 1,0 | 1,0 |
| m-Aminophenol | 0,1 | 0,2 | 0,1 | 0,04 |
| 2-Amino-4-Hydroxyethylaminoanisolsulfat | 0,04 | 0,02 | -- | -- |
| Resorcin | 0,3 | 0,6 | 0,3 | 0,1 |
| 2-Methylresorcin | -- | -- | 0,1 | 0,3 |
| 2-Amino-3-hydroxypyridin | -- | -- | -- | 0,1 |
| Wasser | ad 100 | ad 100 | ad 100 | ad 100 |

### 1.2 Herstellung der Oxidationsmittelzubereitung

| Rohstoff | Menge |
|---|---|
| Natriumbenzoat | 0,04 |
| Dinafriumpyrophosphat | 0,1 |
| Turpinal^{®} SL | 0,3 |
| Kaliumhydroxid 50 % | 0,1 |
| Isopropylmyristat | 0,8 |
| Cetearyl Alcohol | 4,0 |
| Eumulgin^{®} B 2 | 1,0 |
| Bienenwachs | 0,5 |
| Wasserstoffperoxid (50%Ig) | 5,2 |
| Wasser | ad 100 |

### 1.3 Eingesetzte Rohstoffe

| | |
|---|---|
| Cetiol^{®} V | Ölsäuredecylester (INCI-Bezeichnung: Decyl Oleate) (Cognis) |
| Cutina^{®} GMS-SE | INCI-Bezeichnung: Glyceryl Stearate SE (Cognis) |
| Eumulgin^{®} B2 | Cetylstearylalkohol mit ca. 20 EO-Einheiten (INCI-Bezeichnung: Ceteareth-20) (Cognis) |
| Lanette^{®} N | Gemisch aus 90 Gewichststeilen Cetylstearylalkohol und 10 Gewichtsteilen Natriumcetylstearylsulfatl (INCI-Bezeichnung: Cetearyl Alcohol, Sodium Cetearyl Sulfate) (Cognis) |
| Ronacare^{®}Tiliroside | Mischung von Tiliroside und Sorbitol (ca. 4-5Gew% Tiliroside; INCI-Bezeichnung: Sorbitol, Tiliroside) (Merck) |
| Turpinal^{®} SL | 1-Hydroxyethan-1,1-diphosphonsaure (ca. 58 - 61% Aktivsubstanzgehalt; INCI-Bezeichnung: Etidronic Acid, Aqua (Water)) (Solutia) |
| Xanthan^{®} FN | INCI-Bezeichnung: Xanthan Gum (Jungbunzlauer) |

### 2 Ausfärbungen

50g der Farbrezepturen A bis D wurden jeweils mit 100g der Oxidationsmittelzubereitung gemischt Die resultierenden Anwendungszubereitungen wurden jeweils auf eine Strähne Büffelbauchhaar aufgetragen und dort für 30 Minuten bei Raumtemperatur belassen.

Anschließend wurden die Haare gründlich mit einem handelsüblichen Shampoo gespült

## Patentansprüche

1. Mittel zur Farbveränderung keratinischer Fasern, enthaltend in einem kosmetisch akzeptablen Träger mindestens
- mindesten ein Oxidationsfarbstoffvorprodukt vom Entwickler- und/oder Kuppler-Typ;
- Tilirosid.

2. Mittel nach Anspruch 1, **dadurch gekennzeichnet, dass** es weiterhin Ectoin und/oder ein Ectoinderivat enthält.

3. Mittel nach einem der Ansprüche 1 oder 2, **dadurch gekennzeichnet, dass** es weiterhin mindestens eine Esterverbindung, die als Alkoholkomponente einen gesättigten oder ungesättigten, verzweigten oder unverzweigten Wachsalkohol enthält.

4. Verfahren zur Farbveränderung keratinischer Fasern, **dadurch gekennzeichnet dass** ein Mittel nach einem der Ansprüche 1 bis 3 mit einer Oxidationsmittetelzubereitung vermischt wird, die Anwendungszubereitung auf die Fasern aufgetragen wird und nach einer Einwirkzeit wieder abgespült wird.

5. Verfahren zur Farbveränderung keratinischer Fasern, **dadurch gekennzeichnet, dass** in einen ersten Schritt ein Mittel, enthaltend Tilirosid, auf die Fasern aufgetragen wird, nach einer Einwirkzeit ein zweites Mittel, enthaltend mindesten ein Oxidationsfarbstoffvorprodukt vom Entwickler- und/oder Kuppler-Typ, auf die Fasern aufgetragen wird und in einem dritten Schritt die Mittel nach einer weiteren Einwirkzeit abgespült werden.

6. Verfahren zur Farbveränderung keratinischer Fasern, **dadurch gekennzeichnet, dass** in einem ersten Schritt ein Mittel, enthaltend mindesten ein Oxidationsfarbstoffvorprodukt vom Entwickler- und/oder Kuppler-Typ, auf die Fasern aufgetragen wird, nach einer Einwirkzeit ein zweites Mittel, enthaltend Tilirosid, auf die Fasern aufgetragen wird und in einem dritten Schritt die Mittel nach einer weiteren Einwirkzeit abgespült werden.

## Claims

1. Agent for changing the colour of keratinic fibres, said agent comprising in a cosmetically acceptable carrier at least
- at least one oxidation dye precursor of the developer and/or coupler type;
- Tiliroside.

2. Agent according to claim 1, **characterised in that** it further comprises ectoine and/or an ectoine derivative.

3. Agent according to one of claims 1 or 2, **characterised in that** it further comprises at least one ester compound that comprises a saturated or unsaturated, branched or unbranched wax alcohol as the alcohol component.

4. Method for changing the colour of keratinic fibres, **characterised in that** an agent according to one of claims 1 to 3 is blended with an oxidising agent, the application preparation is deposited onto the fibres and after a contact time is rinsed out again.

5. Method for changing the colour of keratinic fibres, **characterised in that** in a first step an agent comprising Tiliroside is deposited onto the fibres, after a contact time a second agent comprising at least one oxidation dye precursor of the developer and/or coupler type is deposited onto the fibres, and in a third step after a further contact time the agents are rinsed out of the fibres.

6. Method for changing the colour of keratinic fibres, **characterised in that** in a first step an agent comprising at least one oxidation dye precursor of the developer and/or coupler type is deposited onto the fibres, after a contact time a second agent comprising Tiliroside is deposited onto the fibres, and in a third step after a further contact time the agents are rinsed out of the fibres.

## Revendications

1. Agent pour changer la couleur de fibres kératiniques, contenant dans un support acceptable du point de vue cosmétique :
- au moins un précurseur de colorant d'oxydation du type développeur et/ou coupleur ;
- du tiliroside.

2. Agent selon la revendication 1, **caractérisé en ce qu'**il contient en outre de l'ectoïne et/ou un dérivé de l'ectoïne.

3. Agent selon l'une quelconque des revendications 1 ou 2, **caractérisé en ce qu'**il contient en outre au moins un composé ester qui contient, à titre de composant alcool un alcool de cire saturé ou insaturé, ramifié ou non ramifié.

4. Procédé pour changer la couleur de fibres kératiniques, **caractérisé en ce qu'**on mélange un agent selon l'une quelconque des revendications 1 à 3 avec une préparation d'agent d'oxydation, on applique sur les fibres la préparation d'application et on l'en élimine à nouveau par rinçage après l'avoir laissé agir pendant un certain temps.

5. Procédé pour changer la couleur de fibres kératiniques, **caractérisé en ce que**, dans une première étape, on applique sur les fibres un agent contenant du tiliroside, après l'avoir laissé agir pendant un certain temps, on applique sur les fibres un deuxième agent contenant au moins un précurseur de colorant d'oxydation du type développeur et/ou coupleur, et dans une troisième étape on élimine l'agent par rinçage après l'avoir laissé agir pendant un laps de temps supplémentaire.

6. Procédé pour changer la couleur de fibres kératiniques, **caractérisé en ce que**, dans une première étape, on applique sur les fibres un agent contenant au moins un précurseur de colorant d'oxydation du type développeur et/ou coupleur, après l'avoir laissé agir pendant un certain temps, on applique sur les fibres un deuxième agent contenant du tiliroside, et dans une troisième étape on élimine l'agent par rinçage après l'avoir laissé agir pendant un laps de temps supplémentaire.
